# EUROPÄISCHE PATENTSCHRIFT

(11) **EP 1 058 524 B1**
(45) Veröffentlichungstag und Bekanntmachung des Hinweises auf die Patenterteilung: **01.09.2004**
(21) Anmeldenummer: 99964652.4
(22) Anmeldetag: 22.12.1999
(51) Int. Cl.: A61F 2/64

(54) **BEINPROTHESE MIT EINEM KÜNSTLICHEN KNIEGELENK UND VERFAHREN ZUR STEUERUNG EINER BEINPROTHESE**
LEG PROSTHESIS WITH AN ARTIFICIAL KNEE JOINT AND METHOD FOR CONTROLLING A LEG PROSTHESIS
PROTHESE DE JAMBE AVEC ARTICULATION DE GENOU ARTIFICIELLE ET PROCEDE DE COMMANDE DE LADITE PROTHESE

(30) Priorität: 24.12.1998 DE 19859931
(43) Veröffentlichungstag der Anmeldung: 13.12.2000
(73) Patentinhaber: Biedermann Motech GmbH, 78054 Villingen-Schwenningen (DE)
(72) Erfinder: BIEDERMANN, Lutz, D-78048 VS-Villingen (DE); MATTHIS, Wilfried, D-79367 Weisweil (DE); SCHULZ, Christian, D-09648 Mittweida (DE)
(74) Vertreter: Prüfer, Lutz H., Dipl.-Phys.
(86) Internationale Anmeldenummer: PCT/EP1999/010280
(87) Internationale Veröffentlichungsnummer: WO 2000/038599

(56) Entgegenhaltungen:
- EP-A- 0 549 855
- EP-A- 0 628 296
- WO-A-99/44547
- DE-A- 19 754 690
- FR-A- 2 623 086
- ARAI K I ET AL: "IRON LOSS OF TERTIARY RECRYSTALLIZED SILICON STEEL" IEEE TRANSACTIONS ON MAGNETICS,US,IEEE INC. NEW YORK, Bd. 25, Nr. 5, 1. September 1989 (1989-09-01), Seiten 3949-3954, XP000069254 ISSN: 0018-9464

## Beschreibung

Die Erfindung betrifft eine Beinprothese mit einem künstlichen Kniegelenk nach dem Oberbegriff des Anspruches 1 und ein Verfahren zur Steuerung einer Beinprothese nach dem Oberbegriff des Patentanspruches 14.

Dokument EP-A-0 549 855 stellt den nächsten Stand der Technik dar.

Beim Laufen mit einer Prothese wird der Prothesenoberschenkel durch den Beinstumpf während des Ganges nach vorn bewegt. Bei nicht angepaßter Dämpfung kann der Unterschenkel sich durch seine Massenträgheit sehr weit anwinkeln. Der Prothesenträger muß dann warten, bis sich die Prothese wieder nach vorn bewegt, bevor er deren Fuß aufsetzen kann. Damit ergibt sich ein unharmonisches Gangbild, ein ungünstiges Zeitverhalten und somit eine schlechte bzw. nicht optimale Trageeigenschaft.

Es sind Beinprothesen mit einem künstlichen Kniegelenk bekannt, bei denen ein Dämpfungselement in Form eines Pneumatik- oder Hydraulikzylinders zur Schwungphasensteuerung und als sogenannte Rückfallbremse vorgesehen ist. Die Anpassung der Beinprothese an den Träger erfolgt dabei mittels eines stationären Ganganalysesystems. Dabei muß der Träger der Prothese einen Testlauf mit der Prothese, beispielsweise auf einem Laufband, ausführen, worauf dann ein Orthopädietechniker eine subjektive Bewertung des Gangbildes vornimmt.
Zusammen mit den subjektiven Empfindungen des Prothesenträgers wird dann eine Anpassung und Einstellung der verschiedenen Bestandteile der Prothese vorgenommen. Das Ergebnis der Einstellung ist oft ungenau, weil die Einstellung mittels subjektiver Kriterien erfolgt. Zudem werden nächträgliche Veränderungen wie die des Gewichtes, der Temperaturen bzw. der Bodenbeschaffenheit nicht berücksichtigt.

Ferner haben die bekannten Dämpfungselemente für künstliche Kniegelenke den Nachteil, daß sie nicht schnell genug auf eine abrupte Änderung der Gangdynamik reagieren können.

Aus der GB 1,191,633 ist eine Beinprothese mit einem künstlichen Kniegelenk mit einer hydraulisch gesteuerten Bremse bekannt, wobei als Hydraulikflüssigkeit eine ferro-kolloidale oder eine andere magnetische Flüssigkeit verwendet wird.

Aus der DE 195 21 464 A1 ist eine Beinprothese mit einem künstlichen Kniegelenk nach dem Oberbegriff des Patentanspruches 1 und ein Verfahren zum Steuern einer solchen nach dem Oberbegriff des Patentanspruches 14 bekannt. Bei dieser Beinprothese kann bei einem Wechsel der Gangart auch die Steuerung der Kniebremse geändert werden und zwar jeweils unter Adaptierung eines gespeicherten Referenzmusters. Verschiedenen Gangarten wie Gehen auf einem ebenen Untergrund oder Treppensteigen ist jeweils ein spezielles Steuerprogramm zur Ansteuerung der Kniebremse zugeordnet. Während des Gehens wird durch Messung der Hüftgelenksmuskelaktivität und Vergleich der Meßwerte mit gespeicherten Referenzwerten die Gangart bestimmt und die Kniebremse mit dem für dieser Gangart zugeordneten Steuerprogramm gesteuert. Die Referenzwerte für eine Gangart werden zuvor für jeden Prothesenträger bestimmt. Eine Änderung eines einer bestimmten Gangart zugeordneten Steuerprogramms selbst erfolgt jedoch nicht.

Bei der bekannten Beinprothese besteht jedoch das Problem, daß eine Anpassung der Prothesensteuerung d.h. der Steuerprogramme an sich ändernde Umstände bezogen auf den Prothesenträger, wie beipielsweise eine Zu- oder Abnahme des Gewichts des Prothesenträgers oder das Tragen anderer Schuhe oder bezogen auf die Umgebung, wie z.B. Gehen in der Ebene auf holprigem Weg, nicht erfolgt.

Aufgabe der Erfindung ist es, eine Beinprothese mit einem künstlichen Kniegelenk und ein Verfahren zum Steuern einer solchen bereitzustellen, die bzw. das einen optimalen an den Träger angepaßten Betrieb der Prothese unabhängig von sich ändernden Betriebsumständen sowie eine schnelle Reaktion auf abrupte Änderungen der Gangdynamik gewährleistet.

Die Aufgabe wird gelöst durch eine Beinprothese gemäß Patentanspruch 1 und durch ein Verfahren zur Steuerung einer solchen nach Patentanspruch 14. Weiterbildungen der Erfindung sind in den Unteransprüchen angegeben.

Weitere Merkmale und Zweckmäßigkeiten der Erfindung ergeben sich aus der Beschreibung von Ausführungsbeispielen anhand der Figuren.

Von den Figuren zeigen:
- Fig. 1:: eine schematische Darstellung einer ersten Ausführung einer Beinprothese mit einem künstlichen Kniegelenk mit einer Sprungphasensteuerung und Rückfallbremse.
- Fig. 2(a):: eine Seitenansicht einer Beinprothese in einer zweiten Ausführungsform;
- Fig. 2(b):: eine Vorderansicht der Beinprothese von Fig. 2(a);
- Fig. 3(a):: eine Seitenansicht einer dritten Ausführungsform einer Beinprothese;
- Fig. 3(b):: eine Vorderansicht der Beinprothese von Fig. 3(a);
- Fig. 4:: eine schematische Darstellung einer Steuer- und Regelvorrichtung für die erfindungsgemäße Beinprothese;
- Fig. 5:: ein Diagramm zur Veranschaulichung der Funktionsweise der Steuerung und Regelung für die erfindungsgemäße Beinprothese und
- Fig. 6:: eine Kurve, die den Kniewinkel in Abhängigkeit von der Zeit für einen Schritt darstellt.

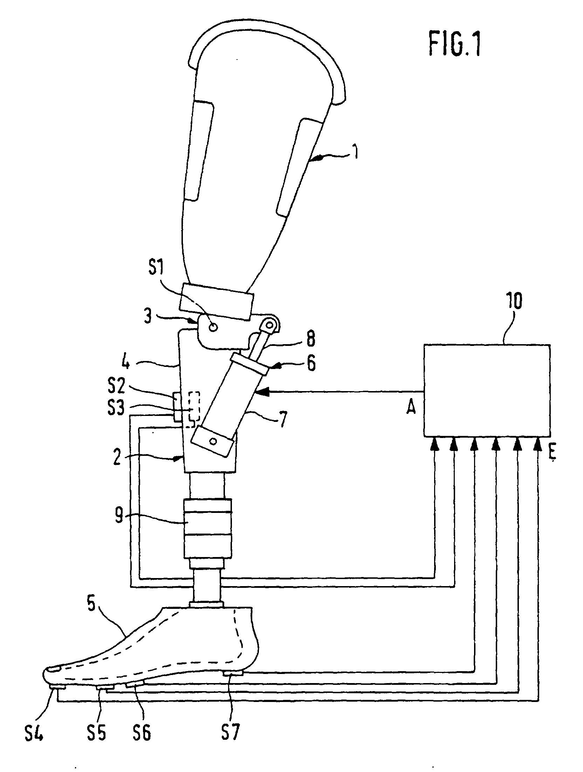

Wie insbesondere aus Fig. 1 ersichtlich ist, umfaßt die Beinprothese in bekannter Weise ein Oberschenkelteil 1 und ein Unterschenkelteil 2 und ein die beiden verbindendes Kniegelenk 3. Das Unterschenkelteil 2 weist ein Schienbeinteil 4 mit einem Unterschenkelrohr 9 und ein mit diesem verbundenes Fußteil 5 auf. Das Fußteil 5 weist in bekannter Weise eine in der Figur nicht dargestellte Blattfeder zum Ermöglichen eines federnden Auftrittes auf. Das Oberschenkelteil 1 ist zum Verbinden mit dem Beinstumpf ausgebildet.

Das Kniegelenk 3 weist ein Dämpfungselement in Form einer hydraulischen Kolben-Zylindereinrichtung 6 auf. Der Zylinder 7 der Kolben-Zylindereinrichtung 6 ist mit dem Schienbeinteil 4 verbunden und die Kolbenstange 8 der Kolben-Zylindereinrichtung 6 ist mit dem Kniegelenk 3 verbunden. Bevorzugt ist der Zylinder 7 ein MRF-Zylinder. Dieser Zylinder ist mit einer magneto-rheologische Flüssigkeit (MR Fluid) gefüllt, die die Eigenschaft aufweist, daß sich unter Einwirkung eines magnetischen Feldes ihre Viskosität im Bereich von etwa 3 bis 5 Millisekunden ändert. Die magneto-rheologische Flüssigkeit besteht aus einer Suspension von magnetisierbaren Teilchen in der Größenordnung von Mikrometern in Öl.

Der Kolben 8 oder der Zylinder 7 der Kolben-Zylindereinrichtung 6 weist ferner einen Elektromagneten auf, der über ein externes Signal ansteuerbar ist und der das Magnetfeld zum Einwirken auf die magneto-rheologische Flüssigkeit bereitstellt.

Die Beinprothese weist ferner eine Anzahl von Sensoren zur Bewegungs- und Kraftmessung auf. Im Kniegelenk 3 ist ein Kniewinkelsensor S1 zur Erfassung des Kniewinkels vorgesehen. Am Schienbeinteil 4 sind optional Beschleunigungssensoren vorgesehen. Ein frontal angeordneter Beschleunigungssensor S2 dient zur Messung der Beschleunigung in Fortbewegungsrichtung, ein seitlich angeordneter Beschleunigungssensor S3 dient zur Messung der Beschleunigung senkrecht zur Fortbewegungsrichtung. Als Beschleunigungssensoren können herkömmliche Beschleunigungssensoren, wie sie beispielsweise aus der Kraftfahrzeugtechnik bekannt sind, verwendet werden.

Zur Messung der auf die Prothese wirkenden Kraft sind ein oder mehrere Kraftsensoren vorgesehen. Gemäß der in Fig. 1 dargestellten Ausführungsform sind im Bereich der Fußsohle Kraftsensoren S4 bis S7 vorgesehen. Der Kraftsensor S4 ist im Zehenbereich angeordnet, die Kraftsensoren S5 und S6 sind im Fußballenbereich angeordnet und ein Kraftsensor S7 ist im Fersenbereich angeordnet. Als Sensoren können herkömmliche Kraftsensoren, beispielsweise solche auf der Basis einer Druckfeder verwendet werden. Die Kraftsensoren liefern Informationen zur Krafteinleitung und ermöglichen die Unterscheidung von Stand- und Schwungphase.

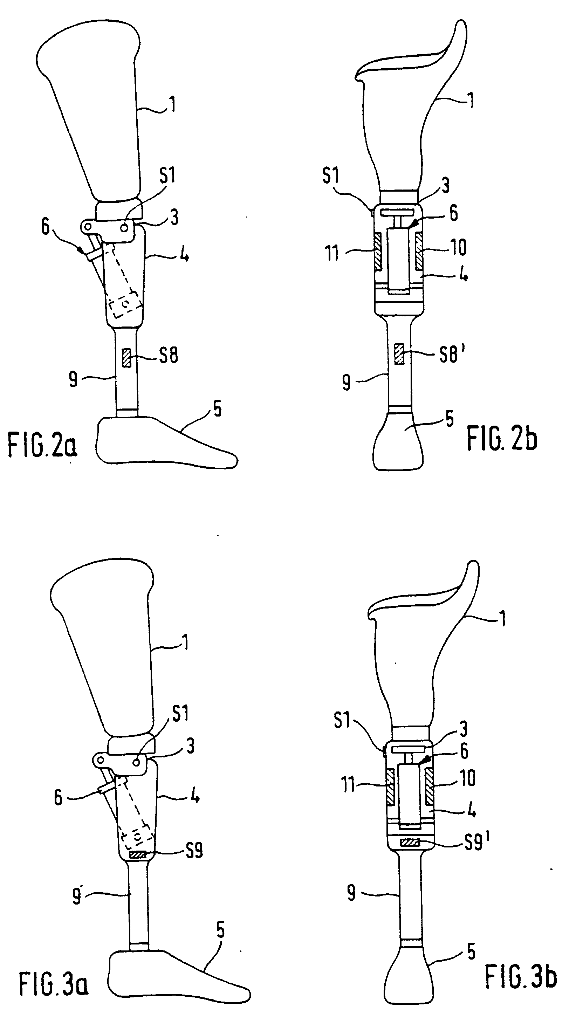

Die Figuren 2(a) und 2(b) zeigen eine bevorzugte Ausführungsform der Beinprothese, bei der Kraftsensoren S8 und S8' am Unterschenkelrohr 9 vorgesehen sind. Die Kraftsensoren sind beispielsweise DMS-Sensoren. Der Kraftsensor S8 ist in Betriebsstellung der Beinprothese am Unterschenkelrohr 9 seitlich entweder innen oder außen vorgesehen und erfaßt die auf die Prothese wirkende Gesamtkraft. Der Kraftsensor S8' ist in Betriebsstellung der Beinprothese am Unterschenkelrohr 9 vorne oder hinten vorgesehen und erfaßt die am Unterschenkelrohr auftretende Biegung.

In den Figuren 3(a) und 3(b) ist eine weitere bevorzugte Ausführungsform der Beinprothese dargestellt, bei der alternativ zur vorbeschriebenen Ausführungsform Kraftsensoren S9 und S9' zur Messung jeweils der Gesamtkraft bzw. der Biegekraft im Schienbeinteil 4 integriert sind. Bevorzugt sind die Kraftsensoren S9, S9' als DMS-Sensoren ausgebildet, die in das Carbon-Trägermaterial des Schienbeinteiles 4 eingebettet sind. Die Anordnung der Sensoren S9 und S9' in Betriebsstellung der Beinprothese ist derart, daß der Sensor S9 seitlich innen oder außen vorgesehen ist und der Sensor S9' hinten oder vorne, analog zu dem Ausführungsbeispiel gemäß den Figuren 2(a) und 2(b).

Die Anordnung der Kraftsensoren im Unterschenkelrohr 9 bzw. im Schienbeinteil 4 hat gegenüber der Anordnung von Kraftsensoren im Fußteil 5 den Vorteil, daß je nach Bedarf unterschiedliche Fußteile verwendet werden können und keine störenden Drähte zur Datenübertragung vom Fußteil zur Steuereinheit der Prothese vorhanden sind.

Die Signalausgänge der Sensoren S1 bis S7 bzw. S1 und S8, 8' oder S9, 9' sind mit einem oder mehreren Eingängen E einer Steuer- bzw. Regeleinheit 10 verbunden. Bevorzugt ist die Steuer- bzw. Regeleinheit 10 wie in den Figuren 2(b) und 3(b) dargestellt ist, in das Schienbeinteil 4 integriert. Ferner ist in das Schienbeinteil 4 oder in das Unterschenkelrohr 9 eine Batterie 11 integriert, die zur Stromversorgung der Steuer- bzw. Regeleinheit 10 dient. Die Steuereinheit weist eine CPU und einen Datenspeicher auf. In dem Datenspeicher ist ein Programm mit einem Algorithmus zur Verarbeitung der eingehenden signale von den Sensoren und zum Erzeugen eines oder mehrerer Ausgangssignale vorgesehen. Ein Signalausgang A der Steuereinheit 10 ist mit der Kolben-Zylindereinrichtung 6 und speziell mit dem in dem Kolben vorgesehenen Elektromagneten verbunden.

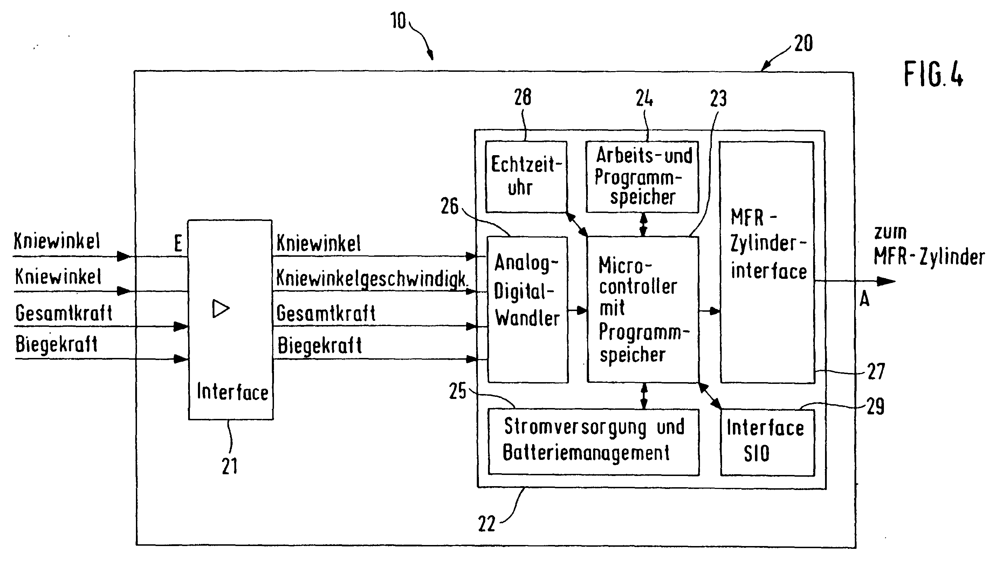

Der Aufbau der Steuer- bzw. Regeleinheit 10 ist aus Fig. 4 ersichtlich und wird anhand dieser näher beschrieben. Die Bauteile zur Verarbeitung der von den Sensoren erzeugten Signale sind auf einer Platine 20 angeordnet. Die Platine 20 umfaßt ein Interface 21 an das die von den Sensoren erfaßten Signale entsprechend dem Kniewinkel, der Gesamtkraft bzw. Bodenreaktionskraft und der Biegekraft angelegt werden. In einer weiteren Ausbildung können auch die Signale der Beschleunigungssensoren eingegeben werden. Das Interface 21 ist derart ausgebildet, daß eine Vorverarbeitung, wie z.B. Verstärkung der Signale stattfindet und eine Kniewinkelgeschwindigkeit berechnet wird. Ferner ist auf der Platine 20 ein elektronischer Schaltkreis 22 vorgesehen, der einen Micro-Controller 23 mit Programmspeicher, einen Arbeits- und Parameterspeicher 24, eine Stromversorgung 25, einen Analog-Digital-Wandler 26 und ein Interface 27 als Schnittstelle zur Kolben-Zylindereinrichtung 6 aufweist. Optional ist eine Echtzeituhr 28 und ein SIO-Interface 29 vorgesehen. Entsprechende Verbindungsleitungen zwischen den einzelnen Bausteinen des Schaltkreises sind vorgesehen. Die im Interface 21 vorverarbeiteten Signale Kniewinkel, Kniewinkelgeschwindigkeit, Gesamtkraft und Biegekraft werden dem Analog-Digital-Wandler 26 zugeführt und die erzeugten digitalen Signale werden dem Micro-Controller 23 zugeführt. Im Micro-Controller 23 ist ein Algorithmus zur vorbestimmten Verarbeitung der Signale abgelegt. Die von dem Micro-Controller abgegebenen Signale werden an das Interface 27 angelegt und zur Steuerung der Kolben-Zylindereinrichtung ausgegeben.

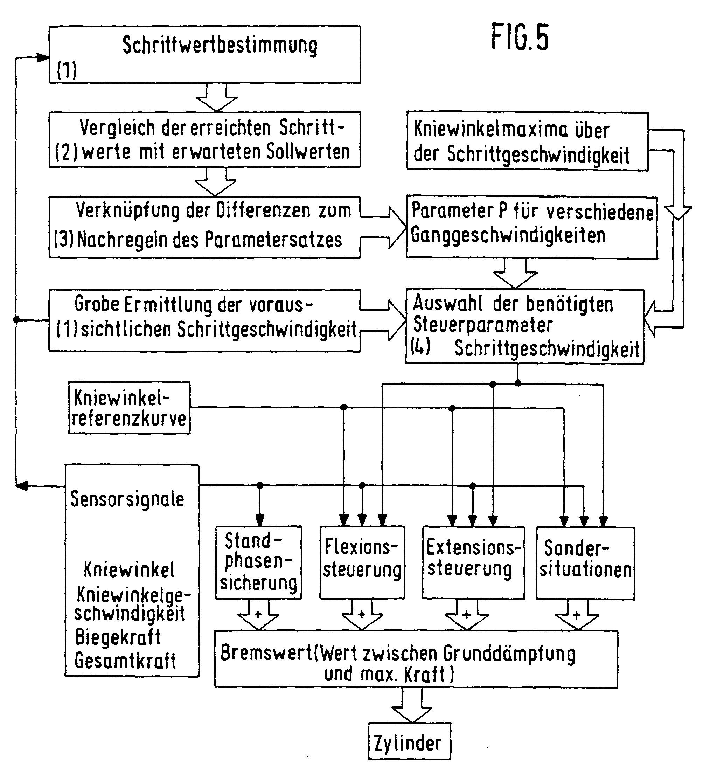

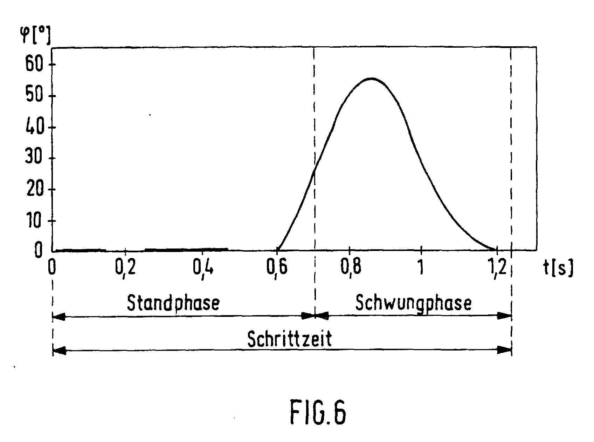

In dem Arbeits- und Parameterspeicher 24 und dem Programmspeicher des Micro- Controllers 23 sind folgende vorgegebene Daten abgelegt:
a) Eine in Fig. 6 dargestellte Kniewinkelreferenzkurve, die die Abhängigkeit des Kniewinkels von der Zeit für eine Schrittzeit darstellt. Die Kniewinkelreferenzkurve setzt sich zusammen aus der Standphase, die die Zeit vom Auftritt mit der Ferse über das Abrollen über den Fußballen bis zum Beginn der Flexion des Kniegelenks umfaßt und eine Schwungphase die die Flexion und die Extension des Kniegelenks bis zum erneuten Auftritt mit der Ferse umfaßt. Die Kniewinkelreferenzkurve gibt ein optimales Schrittverhalten wieder, deren Werte für den Kniewinkel empirisch bestimmt worden sind. Die Kniewinkelreferenzkurve ist für jeden Prothesenträger gleich. Bevorzugt ist die Kniewinkelreferenzkurve normiert, beispielsweise auf 1 Sekunde Schrittzeit, abgelegt.
   Für jede Gangart, d.h. beispielsweise für Gehen auf einer geneigten Ebene, welche das Gehen in der Horizontalen als Sonderfall beinhaltet, oder für Treppensteigen ist eine derartige für alle Prothesenträger gleiche Kniewinkelreferenzkurve abgelegt.
b) eine Zuordnung von Kniewinkelmaxima zu unterschiedlichen Ganggeschwindigkeiten. Diskrete Werte des Kniewinkelmaximum in Bezug auf eine bestimmte Ganggeschwindigkeit sind zuvor empirisch für den jeweiligen Prothesenträger ermittelt worden bzw. sind einfach vorgegeben ohne den aktuellen Prothesenträger zu kennen. Zwischenwerte können durch Interpolation erhalten werden.
c) Steuerparameter P für die Ansteuerung der Kolben-Zylindereinrichtung jeweils für verschiedene Ganggeschwindigkeiten. Die Steuerparameter umfassen einen Extensionsdämpfungsverstärkungsfaktor Ed, der ein Maß für die Dämpfungsverstärkung für die Extension angibt, und einen Flexionsdämpfungsverstärkungsfaktor Fd, der ein Maß für die Dämpfungsverstärkung für die Flexion angibt.
   Diese Steuerparameter P sind zuvor für den jeweiligen Prothesenträger empirisch für verschiedenen Ganggeschwindigkeiten mittels einer Ganganalyse ermittelt worden oder sind einfach als Startwerte für die Ansteuerung der Kolben-Zylindereinrichtung vorgegeben ohne bereits an einen speziellen Prothesenträger angepaßt zu sein. Der in dem Microcontroller 23 abgelegte vorbestimmte Algorithmus zur Steuerung und Regelung der Kolben-Zylindereinrichtung wird anhand von Fig. 5 beschrieben. Der Algorithmus bildet das erfindungsgemäße Verfahren zur Steuerung und Regelung der Steuerung der Beinprothese.

Aus den von dem Analog-Digital-Wandler 26 abgegebenen digitalen Signalen für den Kniewinkel, die Kniewinkelgeschwindigkeit, die Gesamtkraft und die Biegekraft werden im Schritt (1) die folgenden Schrittwerte bestimmt:
- der maximale Flexionswinkel,der dem maximalen Kniewinkel für diesen Schritt entspricht,
- die Streckvoreilzeit, die die Zeit zwischen Extensionsanschlag und Aufsetzen der Ferse ist.
- die Standzeit, und
- die Schrittzeit.
Als Ist-Schrittwerte werden von diesen Schrittwerten der maximale Flexionswinkel und die Streckvoreilzeit definiert.

Gleichzeitig wird im Schritt (1) eine Ermittlung der voraussichtlichen Schrittgeschwindigkeit vorgenommen. Diese erfolgt beispielsweise durch Verknüpfung einer Einleitzeit, die die Zeit ab der Krafteinleitung und ersten Änderung des Kniewinkels bis zum kompletten Abheben vom Boden ist und dem Einleitkniewinkel. Alternativ erfolgt die Bestimmung der Schrittgeschwindigkeit durch Bestimmung der Kniewinkelgeschwindigkeit bei einen bestimten Flexionswinkel, bevorzugt bei etwa bei 20°, bei dem der Fuß ganz vom Boden abgehoben ist.

Im Schritt (2) werden die Ist-Schrittwerte mit Soll-Schrittwerten verglichen. Die Soll-Schrittwerte sind empirisch mittels einer Ganganalyse ermittelte Schrittwerte für eine optimale Protheseneinstellung für ein natürliches Gangverhalten und sind für jeden Prothesenträger gleich. Soll-Schrittwerte sind insbesondere:

Das Kniewinkelmaximum liegt bevorzugt zwischen 55° und 60°. Die Streckvoreilzeit liegt bevorzugt im Bereich von 0,06 bis 0,1 Sekunden.
Die Soll-Schrittwerte für eine bestimmte Gangart entsprechen den aus der Kniewinkelreferenzkurve ermittelten Schrittwerten für diese Gangart. Die Sollschrittwerte beinhalten somit eine Grundform und einen Zeitverlauf, nicht die Absolutwerte.

Im Schritt (3) erfolgt eine Verknüpfung der Differenzen zwischen Soll-Schrittwerten und Ist-Schrittwerten für die ermittelte Schrittgeschwindigkeit in vorbestimmter Weise zur Bestimmung von Korrekturfaktoren für die Steuerparameter P:
a) Liegt der maximale Ist-Flexionswinkel im Bereich des Soll-Kniewinkels, ist keine Korrektur erforderlich. Ist der maximale Ist-Flexionswinkel größer bzw. kleiner als der Soll-Kniewinkel, so ist der Flexionsdämpfungsverstärkungsfaktor Fd zu erhöhen bzw. zu erniedrigen.
b) Ist die Ist-Streckvoreilzeit im Bereich der Soll-Streckvoreilzeit, so ist keine Korrektur erforderlich. Ist die Ist-Streckvoreilzeit größer bzw. kleiner als die Soll-Streckvoreilzeit, so ist der Extensionsdämpfungsverstärkungsfaktor Ed zu erhöhen bzw. zu erniedrigen.
c) Ist die Standzeit größer als 2 Sekunden, so ist der Schritt nicht in die Nachregelung der Steuerparameter einzubeziehen, da er das Loslaufen bzw. Stehenbleiben kennzeichnet.

Das Maß der Erhöhung oder Erniedrigung der Korrekturfaktoren kann dabei betragsmäßig konstant, d.h. unabhängig von der Größe der Differenz zwischen den Ist-Schrittwerten und den Soll-Schrittwerten sein oder es kann abhängig von der Differenz zwischen den Ist-Schrittwerten und den Soll-Schrittwerten sein.

Im Schritt (4) erfolgt eine Auswahl der Steuerparameter P zur Steuerung der Kolben-Zylindereinrichtung in Abhängigkeit von der voraussichtlichen Schrittgeschwindigkeit und dem entsprechenden im Arbeits- und Programmspeicher 24 abgelegten Wert für das Kniewinkelmaximum. Die Steuerparameter P werden entsprechend dem Ergebnis aus dem Schritt (3) mit Korrekturfaktoren verknüpft und somit gegenüber den ursprünglich im Speicher abgelegten, empirisch ermittelten Steuerparametern P für die entsprechende Geschwindigkeit nachgeregelt. Die so erhaltenen korrigierten aktuellen Steuerparameter P' werden wiederum im Speicher als nun gültige Werte abgelegt. und dienen beim nächsten Schritt als Ausgangspunkt für die Nachregelung.

Der Teil des Algorithmus, der die eigentliche Aussteuerung der Kolben-Zylindereinrichtung bewirkt, arbeitet wie folgt:

Die aktuellen Steuerparameter P' werden jeweils zur Flexions- und Extensionsteuerung verwendet, d.h. sie bewirken eine definierte Einstellung der Dämpfung bzw. des Bremswertes der Kolben-Zylindereinrichtung zwischen einer Grunddämpfung und einer maximalen Dämpfung. Die Steuerung der Kolben-Zylindereinrichtung ist für die Kniewinkelreferenzkurve fest vorgegeben. Zur Bestimmung, wann die berechneten korrigierten Steuerparameter P' wirken sollen, wird die Kniewinkelreferenzkurve auf die voraussichtliche Schrittgeschwindigkeit skaliert. Bei der Flexionssteuerung folgt dabei eine Anpassung des maximalen Flexionswinkels und der Flexionszeit, bei der Extensionssteuerung erfolgt eine Anpassung an den maximal erreichten Flexionswinkel und die geforderte Extensionszeit. Die Flexionssteuerung erfolgt nur im Zeitraum zwischen Abstoßen und Erreichen des maximalen Kniewinkels, die Extensionssteuerung erfolgt nur im Zeitraum vom maximalen Kniewinkel bis zum Erreichen des Kniewinkelanschlages. Es wird eine Differenzbildung zwischen der aktuellen Kniewinkelgeschwindigkeit zur benötigten Geschwindigkeit, um die nächste Kniewinkelposition im Zeitbereich zu erreichen, durchgeführt. Ist die aktuelle Kniewinkelgeschwindigkeit zu groß, muß eine Dämpfung erfolgen. Im Falle der Flexion ist die Dämpfung gleich der Geschwindigkeitsdifferenz mal dem Flexionsdämpfungsverstärkungsfaktor. Im Falle der Extension ist die Dämpfung gleich der Geschwindigkeitsdifferenz mal dem Extensionsdämpfungsverstärkungsfaktor. Ist die aktuelle Geschwindigkeit zu klein, erfolgt keine Dämpfung.

Wie aus Fig. 5 ersichtlich ist, stellt der Teilschritt von Schritt (1), in dem die voraussichtliche Schrittgeschwindigkeit bestimmt wird, und der Schritt (4) mit der nachfolgenden Ansteuerung der Kolben-Zylindereinrichtung die Steuerebene dar. Der Teilschritt (1) in dem die Schrittwerte bestimmt werden zusammen mit den Schritten (2) und (3) stellt die Regelebene dar.

Die Steuerung und Regelung über den Algorithmus beinhaltet auch eine Standphasensicherung. Hierzu werden die Signale Gesamtkraft und Biegekraft verwendet. Ist im Auftrittszeitraum die Gesamtkraft ansteigend und die Biegung im Fersenbereich oder steigt der Kniewinkel an und die Gesamtkraft ist auf dem Fuß, während die Biegekraft nicht im Vorfußbereich ist, so wird die Bremse zu 100% aktiviert, d.h. es kann keine weitere Flexion erfolgen. Ist jedoch die Biegekraft im Vorfußbereich, so wird die Bremse nicht aktiviert.

Die Steuerung von Sondersituationen umfaßt die Steuerung Treppe steigen, stolpern und stürzen. Gehen auf geneigter Ebene wird wie die Extension bzw. Flexion behandelt. Für die Sondersituation Treppen steigen
gibt es gesonderte empirische Werte, d.h. eine spezielle Kniewinkelreferenzkurve. Für die Sondersituation Stolpern oder Stürzen oder Anstoßen wird die Standphasensicherung aktiviert.

Im Betrieb arbeitet die Steuerung der Beinprothese wie folgt. Die Meßdaten der Kniewinkel- und Kraftsensoren werden an die Steuereinheit 10 geleitet. In Abhängigkeit von den Meßdaten werden durch die Steuereinheit 10 Steuersignale für die Kolben-Zylindereinrichtung erzeugt und an diese geleitet. In Abhängigkeit von den Steuersignalen wird von dem Elektromagneten ein definiertes Magnetfeld erzeugt, welches eine bestimmte Viskositätsänderung der magneto-rheologischen Flüssigkeit in dem Zylinder 7 hervorruft. Durch die Änderung der Viskosität kann die Eintauchtiefe des Kolbens 8 in den Zylinder 7 und damit die Dämpfung entsprechend gesteuert werden. Die Änderung der Dämpfung erfolgt dabei innerhalb einer Zeitspanne von etwa 3 bis 5 Millisekunden. Dies ist insbesondere vorteilhaft beim Einsatz der Dämpfung als Rückfallbremse. Wenn der Träger der Beinprothese stolpert, so kann durch die sich unmittelbar aufbauende Dämpfung ein Einklappen des Unterschenkelteils frühzeitig verhindert werden.

Die Steuereinheit, die Sensoren und das Dämpfungselement sind, wie beschrieben, in einem Regelkreis miteinander verbunden, d.h. es erfolgt eine Einstellung der Dämpfung während des Gehens durch Nachregelung der Steuerparameter P für die Einstellung der Dämpfung ausgehend von vorgegebenen Werten für die Steuerparameter P.Dies hat gegenüber einer herkömmlichen Prothesensteuerung den Vorteil, daß die Einstellung der Prothesenfunktionen unmittelbar in Abhängigkeit von dem natürlichen Gangverhalten des Prothesenträgers erfolgt.

Insbesondere hat die beschriebene Beinprothese unter Verwendung des beschriebenen Algorithmus den Vorteil, daß die Prothese nicht unter Auswahl eines definierten Gangverhaltens aus vielen zuvor ermittelten Gangverhalten für einen Prothesenträger angesteuert wird, sondern daß eine Nachregelung der vorbestimmten Werte für ein definiertes Gangverhalten erfolgt. Die Prothese paßt sich damit flexibel dem augenblicklichen Gangverhalten an und es ist ein nahezu natürlicher Gang möglich. Außerdem wird durch die Nachregelung der Steuerparameter für verschiedene Geschwindigkeiten eine Anpassung der Prothesensteuerung an geänderte Umstände, wie beispielsweise geändertes Gewicht des Prothesenträgers oder die Verwendung eines anderen Fußteiles oder eines anderen Schuhes ermöglicht.

Abgewandelte Ausführungsformen sind denkbar. Es können weniger oder mehr als die oben beschriebenen Sensoren vorgesehen sein.

Anstelle einer Kolben-Zylindereinrichtung mit einem in dem Zylinder axial verschiebbaren Kolben kann auch eine Kolben-Zylindereinrichtung mit einem Drehkolben verwendet werden, der beispielsweise mit Schaufeln versehen ist, die in Abhängigkeit von der Viskosität der magneto-rheologischen Flüssigkeit einen bestimmten Widerstand im Zylinder erfahren. Die Kolbenstange ist dabei mit einer Drehwelle des Kniegelenks verbunden.

Die Erfindung ist jedoch nicht beschränkt auf eine Beinprothese mit einem Hydraulikzylinder mit einer magneto-rheologischen Flüssigkeit als Dämpfungselement, sondern es können auch herkömmliche Hydraulikzylinder eingesetzt werden, bei denen sich die Dämpfung über ein Bypassventil zwischen den Kammern einstellen läßt. Die Steuerung der Ventilöffnungerfolgt dann beispielsweise über Schrittmotoren.

## Patentansprüche

1. Beinprothese mit
einem Oberschenkelteil (1), einem Unterschenkelteil (2) und einem die beiden verbindenden Kniegelenk (3), wobei das Kniegelenk (3) ein Dämpfungselement (6) zum Steuern der Kniegelenksbewegung aufweist,
einer Einrichtung (S1) zum Erfassen des Kniewinkels,
einer Einrichtung (S4-S7; S8, S8', S9, S9') zum Erfassen der auf die Prothese wirkenden Kraft,
einer Steuerung (10) zum Steuern des Dämpfungselements (6) in Abhängigkeit von den erfassten Werten für den Kniewinkel und für die Kraft, und
einer Regelvorrichtung (10), die in Abhängigkeit von erfassten Werten für den Kniewinkel und die Kraft die Steuerung des Dämpfungselements entsprechend dem Gangverhalten regelt, wobei
die Steuerung (10) des Dämpfungselements derart ausgebildet ist, dass sie das Dämpfungselement (6) in Abhängigkeit von zuvor für verschiedene Ganggeschwindigkeiten gespeicherten Steuerparametern (P) steuert, und
**dadurch gekennzeichnet, dass** die Regelvorrichtung (10) derart ausgebildet ist, dass sie die Parameter in Abhängigkeit von dem Gangverhalten nachregelt, indem sie
als Funktion der erfassten Werte für den Kniewinkel und für die Kraft einen Steuerparameter (P) aus den gespeicherten Steuerparametern auswählt,
den ausgewählten Steuerparameter (P) als Funktion der erfassten Werte für den Kniewinkel und für die Kraft auf einen Wert (P') ändert, der gleich groß wie oder anders als der vorher gespeicherte Wert (P) ist, und
den geänderten Steuerparameter (P') an Stelle des ausgewählten Steuerparameters (P) speichert.

2. Beinprothese nach Anspruch 1, **dadurch gekennzeichnet, dass** die Regelvorrichtung (10) derart ausgebildet ist, dass eine Nachregelung unter der Bedingung erfolgt, dass ermittelte Ist-Schrittwerte von vorgegebenen Soll-Schrittwerten, die für jeden Prothesenträger gleich sind, abweichen.

3. Beinprothese nach Anspruch 2, **dadurch gekennzeichnet, dass** die Soll-Schrittwerte definiert sind als ein maximaler Kniewinkel und eine Streckvoreilzeit, die die Zeit zwischen einem Extensionsanschlag und einem Aufsetzen der Ferse innerhalb eines Schrittes ist.

4. Beinprothese nach Anspruch 3, **dadurch gekennzeichnet, dass** der maximale Kniewinkel im Bereich zwischen 55° und 60° liegt.

5. Beinprothese nach Anspruch 3 oder 4, **dadurch gekennzeichnet, dass** die Streckvoreilzeit im Bereich von 0,06 bis 0,1 Sekunden liegt.

6. Beinprothese nach einem der Ansprüche 1 bis 5, **dadurch gekennzeichnet, dass** die Steuerparameter (P) einen Extensionsdämpfungsverstärkungsfaktor (Ed) und einen Flexionsdämpfungsverstärkungsfaktor (Fd) beinhalten, in Abhängigkeit von welchen das Dämpfungselement (6) jeweils für den Fall der Flexion oder der Extension auf einen bestimmten Dämpfungswert eingestellt wird.

7. Beinprothese nach einem der Ansprüche 1 bis 6, **dadurch gekennzeichnet, dass** das Dämpfungselement (6) als MRF-Zylinder (magneto-rheologischer Fluid-Zylinder) ausgebildet ist.

8. Beinprothese nach einem der Ansprüche 1 bis 7, **dadurch gekennzeichnet, dass** die Einrichtung zum Erfassen des Kniewinkels einen im Kniegelenk vorgesehenen Kniewinkelsensor (S1) umfasst.

9. Beinprothese nach einem der Ansprüche 1 bis8, **dadurch gekennzeichnet, dass** die Einrichtung (S4 bis S7; S8, S8', S9, S9') zum Erfassen der auf die Prothese wirkenden Kraft eine Einrichtung zum Erfassen der Gesamtkraft und eine Einrichtung zum Erfassen der Biegekraft aufweist.

10. Beinprothese nach einem der Ansprüche 1 bis9, **dadurch gekennzeichnet, dass** das Unterschenkelteil (2) ein Schienbeinteil (4) und ein Unterschenkelrohr (9) aufweist und dass die Einrichtung zum Erfassen der Kraft wenigstens zwei im Unterschenkelrohr vorgesehene Kraftsensoren (S8, S8') aufweist.

11. Beinprothese nach einem der Ansprüche 1 bis 9, **dadurch gekennzeichnet, dass** das Unterschenkelteil (2) ein Schienbeinteil (4) aufweist, und dass wenigstens zwei Kraftsensoren (S9, S9') in Form von DMS-Sensoren in das Material des Schienbeinteiles integriert sind.

12. Beinprothese nach einem der Ansprüche 1 bis11, **dadurch gekennzeichnet, dass** die Regelvorrichtung (10) am Unterschenkelteil (2) der Beinprothese vorgesehen ist.

13. Verfahren zum Steuern einer Beinprothese mit einem Oberschenkelteil (1), einem Unterschenkelteil (2) und einem die beiden verbindenden Kniegelenk (3), wobei das Kniegelenk (3) ein Dämpfungselement (6) zum Steuern der Kniegelenksbewegung aufweist, und mit einer Einrichtung (S1) zum Erfassen des Kniewinkels und einer Einrichtung (S4-S7; S8, S8'; S9, S9') zum Erfassen der auf die Prothese wirkenden Kraft, wobei das Dämpfungselement (6) in Abhängigkeit von erfassten Werten für den Kniewinkel und für die Kraft in Abhängigkeit von zuvor für den jeweiligen Prothesenträger für verschiedene Ganggeschwindigkeiten gespeicherten Steuerparametern (P) in Abhängigkeit von der Schrittgeschwindigkeit gesteuert wird,
**dadurch gekennzeichnet, dass**
die Steuerparameter (P) angepasst an das Gangverhalten nachgeregelt werden, indem
als Funktion der erfassten Werte für den Kniewinkel und für die Kraft ein Steuerparameter (P) aus den gespeicherten Steuerparametern ausgewählt wird,
der ausgewählte Steuerparameter (P) als Funktion der erfassten Werte für den Kniewinkel und für die Kraft auf einen Wert (P') geändert wird, der gleich groß wie oder anders als der vorher gespeicherte Wert (P) ist, und
der geänderte Steuerparameter (P') an Stelle des ausgewählten Steuerparameters (P) gespeichert wird.

14. Verfahren zum Steuern einer Beinprothese nach Anspruch 13, **dadurch gekennzeichnet, dass** während jeden Schrittes mit der Prothese die folgenden Verfahrensschritte ausgeführt werden:
a) Bestimmen einer voraussichtlichen Schrittgeschwindigkeit;
b) Bestimmen von Ist-Schrittwerten, die das augenblickliche Gangverhalten kennzeichnen;
c) Vergleich der Ist-Schrittwerte mit vorgegebenen Soll-Schrittwerten;
d) Ändern der Steuerparameter (P) in Abhängigkeit von dem Ergebnis des Vergleichs der Ist-Schrittwerte mit den Soll-Schrittwerten.

15. Verfahren zum Steuern einer Beinprothese nach Anspruch 14, **dadurch gekennzeichnet, dass** die Soll-Schrittwerte den maximalen Kniewinkel und die Streckvoreilzeit umfassen, wobei die Streckvoreilzeit die Zeit zwischen einem Extensionsanschlag und einem Aufsetzen der Ferse ist, und wobei die Bereiche für die Soll-Schrittwerte für jeden Prothesenträger gleich sind.

16. Verfahren zum Steuern einer Beinprothese nach Anspruch 15, **dadurch gekennzeichnet, dass** der maximale Kniewinkel im Bereich von 55° bis 60 ° liegt und die Streckvoreilzeit im Bereich von 0,06 bis 0,1 Sekunden liegt.

17. Beinprothese nach einem der Ansprüche 1 bis 12, **dadurch gekennzeichnet, dass** die Regelvorrichtung (10) so ausgebildet ist, dass sie ausgehend von den jeweils augenblicklich gültigen Werten für die Steuerparameter (P) innerhalb einer Schrittperiode die Steuerparameter (P) in Abhängigkeit von den erfassten Werten für den Kniewinkel und die Kraft derart ändert, dass eine Differenz von den geänderten Werten für die Steuerparameter (P') zu fest vorgegebenen zuvor gespeicherten Steuerparametern, die einem optimalen Gangverhalten entsprechen, minimiert wird.

18. Beinprothese nach einem der Ansprüche 2 bis 12, **dadurch gekennzeichnet, dass** die Regelvorrichtung (10) so ausgebildet ist, dass sie ausgehend von den jeweils augenblicklich gültigen Werten für die Steuerparameter (P) innerhalb einer Schrittperiode die Steuerparameter (P) in Abhängigkeit von den erfassten Werten für den Kniewinkel und die Kraft derart ändert, dass eine Differenz zwischen den ermittelten Ist-Schrittwerten und vorgegebenen Soll-Schrittwerten, die einem optimalen Gangverhalten entsprechen, minimiert wird.

## Claims

1. Leg prosthesis with
a thigh part (1), a below-knee part (2), and a knee joint (3) connecting the two, said knee joint (3) having a damping element (6) for controlling the movement of the knee joint,
a device (S1) for detecting the knee angle,
a device (S4-S7; S8, S8', S9, S9') for detecting the force acting on the prosthesis,
a control unit (10) for controlling the damping element (6) as a function of the detected values for the knee angle and for the force, and
a regulating device (10) which, as a function of the detected values for the knee angle and the force, regulates the control of the damping element in accordance with the gait pattern,
the control unit (10) of the damping element being configured in such a way that it controls the damping element (6) as a function of control parameters (P) which have been stored in advance for different walking speeds, and
**characterized in that** the regulating device (10) is configured in such a way that it readjusts the parameters as a function of the gait pattern, by selecting a control parameter (P) from the stored control parameters as a function of the detected values for the knee angle and for the force,
by changing the selected control parameter (P), as a function of the detected values for the knee angle and for the force, to a value (P') which is the same as or different than the previously stored value (P), and
by storing the changed control parameter (P') instead of the selected control parameter (P).

2. Leg prosthesis according to Claim 1, **characterized in that** the regulating device (10) is configured in such a way that readjustment takes place on condition that actual step values detected deviate from predetermined reference step values, which are identical for each prosthesis wearer.

3. Leg prosthesis according to Claim 2, **characterized in that** the reference step values are defined as a maximum knee angle and a lead-in time, which is the time between an extension stop and touchdown of the heel within one step.

4. Leg prosthesis according to Claim 3, **characterized in that** the maximum knee angle lies in the range between 55° and 60°.

5. Leg prosthesis according to Claim 3 or 4, **characterized in that** the lead-in time lies in the range of 0.06 to 0.1 seconds.

6. Leg prosthesis according to one of Claims 1 to 5, **characterized in that** the control parameters (P) include an extension damping intensification factor (Ed) and a flexion damping intensification factor (Fd), as a function of which the damping element (6) is set to a defined damping value for the case of flexion or extension.

7. Leg prosthesis according to one of Claims 1 to 6, **characterized in that** the damping element (6) is designed as an MRF cylinder (magneto-rheological fluid cylinder).

8. Leg prosthesis according to one of Claims 1 to 7, **characterized in that** the device for detecting the knee angle comprises a knee angle sensor (S1) provided in the knee joint.

9. Leg prosthesis according to one of Claims 1 to 8, **characterized in that** the device (S4 to S7; S8, S8', S9, S9') for detecting the force acting on the prosthesis has a device for detecting the total force and a device for detecting the bending force.

10. Leg prosthesis according to one of Claims 1 to 9, **characterized in that** the below-knee part (2) has a tibial part (4) and a below-knee tube (9), and **in that** the device for detecting the force has at least two force sensors (S8, S8') provided in the below-knee tube.

11. Leg prosthesis according to one of Claims 1 to 9, **characterized in that** the below-knee part (2) has a tibial part (4), and **in that** at least two force sensors (S9, S9') in the form of DMS sensors are integrated into the material of the tibial part.

12. Leg prosthesis according to one of Claims 1 to 11, **characterized in that** the regulating device (10) is provided on the below-knee part (2) of the leg prosthesis.

13. Method for controlling a leg prosthesis with a thigh part (1), a below-knee part (2), and a knee joint (3) connecting the two, said knee joint (3) having a damping element (6) for controlling the movement of the knee joint, and with a device (S1) for detecting the knee angle and a device (S4-S7; S8, S8', S9, S9') for detecting the force acting on the prosthesis, the damping element (6) being controlled as a function of detected values for the knee angle and for the force as a function of control parameters (P) which have been stored in advance for the respective prosthesis wearer for different walking speeds, as a function of the step speed,
**characterized in that**
the control parameters (P) are readjusted to the gait pattern
by selecting a control parameter (P) from the stored control parameters as a function of the detected values for the knee angle and for the force,
by changing the selected control parameter (P), as a function of the detected values for the knee angle and for the force, to a value (P') which is the same as or different than the previously stored value (P), and
by storing the changed control parameter (P') instead of the selected control parameter (P).

14. Method for controlling a leg prosthesis according to Claim 13, **characterized in that**, during each step with the prosthesis, the following method steps are carried out:
a) determination of an anticipated step speed;
b) determination of actual step values which characterize the instantaneous gait pattern;
c) comparison of the actual step values with predetermined reference step values;
d) changing of the control parameters (P) as a function of the result of the comparison of the actual step values with the reference step values.

15. Method for controlling a leg prosthesis according to Claim 14, **characterized in that** the reference step values include the maximum knee angle and the lead-in time, said lead-in time being the time between an extension limit and touchdown of the heel, and the ranges for the reference step values being identical for each prosthesis wearer.

16. Method for controlling a leg prosthesis according to Claim 15, **characterized in that** the maximum knee angle lies in the range of 55° to 60°, and the lead-in time lies in the range of 0.06 to 0.1 seconds.

17. Leg prosthesis according to one of Claims 1 to 12, **characterized in that** the regulating device (10) is configured in such a way that, starting out from the respective instantaneously valid values for the control parameters (P), it changes the control parameters (P), within one step period, as a function of the detected values for the knee angle and the force, so that a difference between the changed values for the control parameters (P') and invariably predetermined control parameters which have been stored in advance, and which correspond to an optimal gait pattern, is minimized.

18. Leg prosthesis according to one of Claims 2 to 12, **characterized in that** the regulating device (10) is configured in such a way that, starting out from the respective instantaneously valid values for the control parameters (P), it changes the control parameters (P), within one step period, as a function of the detected values for the knee angle and the force, so that a difference between the detected actual step values and predetermined reference step values, which correspond to an optimal gait pattern, is minimized.

## Revendications

1. Prothèse de membre inférieur comportant une partie de jambe (1), une partie de cuisse (2) et une articulation de genou (3) reliant ces deux parties, l'articulation de genou (3) comportant un élément amortisseur (6) pour régler automatiquement le mouvement de l'articulation de genou, un dispositif (S1) d'acquisition de l'angle de genou, un dispositif (S4-S7 ; S8, S8', S9, S9') d'acquisition de la force s'exerçant sur le genou, un élément de réglage automatique (10) pour régler automatiquement l'élément amortisseur (6) en fonction des valeurs d'angle de genou et de force saisies, et un dispositif de réglage (10) qui, en fonction de l'angle de genou et de la force exercée, règle l'élément de réglage automatique de l'élément amortisseur selon le comportement de marche, le dispositif de réglage automatique (10) de l'élément amortisseur étant conçu de manière à régler automatiquement l'élément amortisseur (6) en fonction de paramètres de contrôle (P) préalablement enregistrés pour différentes vitesses de marche, **caractérisée par le fait que** le dispositif de réglage (10) est conçu de manière à réaliser un réglage d'ajustement des paramètres en fonction du comportement de marche en sélectionnant, parmi les paramètres de contrôle enregistrés, un paramètre de contrôle (P) en fonction des valeurs d'angle de genou et de force saisies, en donnant au paramètre de contrôle (P) sélectionné une valeur modifiée (P') qui dépend des valeurs d'angle de genou et de force saisies et qui est identique à ou différente de celle préalablement enregistrée, et en enregistrant la valeur modifiée (P') du paramètre de contrôle à la place de celle du paramètre de contrôle sélectionné (P).

2. Prothèse de membre inférieur selon la revendication 1, **caractérisée par le fait que** le dispositif de réglage (10) est conçu de manière à réaliser un réglage d'ajustement lorsque des valeurs réelles de pas s'écartent de valeurs de consigne, identiques pour chaque porteur de prothèse.

3. Prothèse de membre inférieur selon la revendication 2, **caractérisée par le fait que** les valeurs de consigne de pas sont définies comme étant la valeur de l'angle de genou maximal et celle de la durée d'atterrissage, la durée d'atterrissage étant la durée entre la butée de l'extension et la pose du talon, pour chaque pas.

4. Prothèse de membre inférieur selon la revendication 3, **caractérisée par le fait que** l'angle de genou maximal est compris entre 55 ° et 60 °.

5. Prothèse de membre inférieur selon la revendication 3 ou 4, **caractérisée par le fait que** la durée d'atterrissage est comprise entre 0,06 et 0,1 seconde.

6. Prothèse de membre inférieur selon l'une des revendications 1 à 5, **caractérisée par le fait que** les paramètres de contrôle (P) comprennent un facteur amplificateur d'amortissement en extension (Ed) et un facteur amplificateur d'amortissement en flexion (Fd), qui déterminent le réglage de l'indice d'amortissement de l'élément amortisseur respectivement en flexion ou en extension.

7. Prothèse de membre inférieur selon l'une des revendications 1 à 6, **caractérisée en ce que** l'élément amortisseur (6) est un cylindre FMR (cylindre à fluide magnéto-rhéologique).

8. Prothèse de membre inférieur selon l'une des revendications 1 à 7, **caractérisée par le fait que** le dispositif d'acquisition de l'angle de genou comprend un capteur d'angle de genou (S1) dans l'articulation de genou.

9. Prothèse de membre inférieur selon l'une des revendications 1 à 8, **caractérisée par le fait que** le dispositif (S4 à S7 ; S8, S8', S9, S9') d'acquisition de la force s'exerçant sur la prothèse comprend un dispositif d'acquisition de la force globale et un dispositif d'acquisition de la force de fléchissement.

10. Prothèse de membre inférieur selon l'une des revendications 1 à 9, **caractérisé par le fait que** la partie de jambe (2) comporte une partie de tibia (4) et une partie de jambe tubulaire (9) et que le dispositif d'acquisition de la force comprend au moins deux capteurs de force (S8, S8') situés dans la partie de jambe tubulaire (9).

11. Prothèse de membre inférieur selon l'une des revendications 1 à 9, **caractérisée par le fait que** la partie de jambe (2) comprend une partie de tibia (4) et qu'au moins deux capteurs de force (S9, S9') qui sont des capteurs extensométriques, sont intégrés dans le matériau de la partie de tibia.

12. Prothèse de membre inférieur selon l'une des revendications 1 à 11, **caractérisée par le fait que** le dispositif de réglage (10) est situé au niveau de la partie de jambe (2) de la prothèse de membre inférieur.

13. Procédé de réglage automatique d'une prothèse de membre inférieur avec une partie de cuisse (1), une partie de jambe (2) et une articulation de genou (3) reliant ces deux premières parties, l'articulation de genou (3) comportant un élément amortisseur (6) pour le réglage automatique du mouvement de l'articulation de genou, et avec un dispositif (S1) d'acquisition de la valeur de l'angle de genou et un dispositif (S4-S7 ; S8, S8' ; S9, S9') d'acquisition de la valeur de la force s'exerçant sur la prothèse, l'élément amortisseur (6) étant réglé automatiquement en fonction de la vitesse de marche, en fonction des valeurs d'angle de genou et de force saisies et en fonction de paramètres de contrôle (P) préalablement enregistrés pour chaque porteur de prothèse et pour différentes vitesse de marche, **caractérisé par le fait que** les paramètres de contrôle (P) sont modifiés par un réglage d'ajustement, le réglage d'ajustement comprenant la sélection, parmi les paramètres de contrôle enregistrés, d'un paramètre de contrôle (P) en fonction des valeurs d'angle de genou et de force saisies, la modification du paramètre de contrôle (P) sélectionné à une valeur modifiée (P') qui dépend des valeurs d'angle de genou et de force saisies et qui est identique à ou différente de celle préalablement enregistrée, et l'enregistrement de la valeur modifiée (P') du paramètre de contrôle à la place de celle du paramètre de contrôle sélectionné (P).

14. Procédé de réglage automatique d'une prothèse de membre inférieur selon la revendication 13, **caractérisé par le fait que**, à chaque pas fait avec la prothèse, se déroule la suite d'étapes suivantes :
(a) détermination d'une vitesse de pas prévisible,
(b) détermination de la vitesse de pas réelle caractérisant le comportement de marche instantané,
(c) comparaison des valeurs réelles de pas avec des valeurs de consigne prédéterminées, et
(d) ajustement des paramètres de contrôle (P) en fonction du résultat de la comparaison des valeurs réelles de pas avec les valeurs de consigne de pas.

15. Procédé de réglage automatique d'une prothèse de membre inférieur selon la revendication 14, **caractérisé par le fait que** les valeurs de consigne de pas comprennent la valeur de l'angle de genou maximal et celle de la durée d'atterrissage, la durée d'atterrissage étant la durée entre la butée de l'extension et la pose du talon, les intervalles des valeurs de consigne de pas étant identiques pour chaque porteur de prothèse.

16. Procédé de réglage automatique d'une prothèse de membre inférieur selon la revendication 15, **caractérisé par le fait que** l'angle maximal de genou est compris entre 55 ° et 60 ° et que la durée d'atterrissage est comprise entre 0,06 et 0,1 seconde.

17. Prothèse de membre inférieur selon l'une des revendications 1 à 12, **caractérisée par le fait que** le dispositif de réglage (10) est conçu de manière modifier les paramètres de contrôle (P), à l'intérieur d'une période de pas, en partant des valeurs actuellement valables des paramètres de contrôle (P) et en fonction des valeurs d'acquisition de l'angle de genou et de la force exercée, de sorte que la différence entre les valeurs modifiées des paramètres de contrôle (P') et les paramètres de contrôle fixes, préalablement enregistrés, correspondant à un comportement de marche optimal, soit minimale.

18. Prothèse de membre inférieur selon l'une des revendications 2 à 12, **caractérisée par le fait que** le dispositif de réglage (10) est conçu de manière à modifier les paramètres de contrôle (P), à l'intérieur d'une période de pas, en partant des valeurs actuellement valables des paramètres de contrôle (P) et en fonction des valeurs d'acquisition de l'angle de genou et de la force exercée, de sorte que la différence entre les valeurs réelles de pas mesurées et les valeurs de consigne de pas imposées, correspondant à un comportement de marche optimal, soit minimale.
